# EUROPÄISCHE PATENTANMELDUNG

(11) **EP 1 233 009 A2**
(43) Veröffentlichungstag der Anmeldung: **21.08.2002**
(21) Anmeldenummer: 02002124.2
(22) Anmeldetag: 29.01.2002
(51) Int. Cl.: C07B 37/04, C07C 25/18, C07C 209/68, C07C 231/14, C07C 241/02, C07C 253/00

(54) **Verfahren zur Herstellung von 4-substituierten 2-Alkyl- und 2-Alkoxylbiphenylen**

(30) Priorität: 16.02.2001 DE 10107227
(71) Anmelder: Clariant GmbH, 65929 Frankfurt am Main (DE)
(72) Erfinder: Meudt, Andreas, Dr., 65439 Flörsheim-Weilbach (DE); Scherer, Stefan, Dr., 64572 Büttelborn (DE); Vollmüller, Frank, Dr., 60323 Frankfurt am Main (DE); Kautz, Heinz Gorg, 63633 Bierstein (DE)
(74) Vertreter: Hütter, Klaus, Dr.

(57) **Zusammenfassung**

Verfahren zur Herstellung von 4-substituierten 2-Alkyl- und 2-Alkoxybiphenylen der Formel (I), 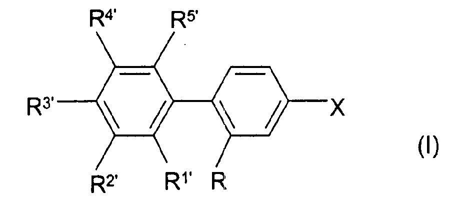 worin die Substituenten X, R und R^{1'} bis R^{5'} die folgende Bedeutung haben:
X steht für NH₂, NHAc, NH(C=O)R¹ mit R¹ = geradkettiges oder verzweigtes C₁- bis C₈-Alkyl, Fluor, Chlor, Brom, Iod, N₂H₃ oder Cyanid,
R steht für geradkettiges oder verzweigtes C₁- bis C₈-Alkyl oder C₁- bis C₅-Alkoxy und
R^{1'} bis R^{5'} stehen unabhängig voneinander für H, CH₃, geradkettiges oder verzweigtes C₁- bis C₈-Alkyl, F, Cl, CN, NO₂, CHO, COOH, CH₂OH, C(=O)CH₃, C₁- bis C₅-Alkoxy,
durch Umsetzung von Phenylboronsäuren der Formel (II) mit 4-Brom- oder 4-lodalkyl- oder alkoxyanilinen oder -aniliden der Formel (III) zu Verbindungen der Formel (IV), 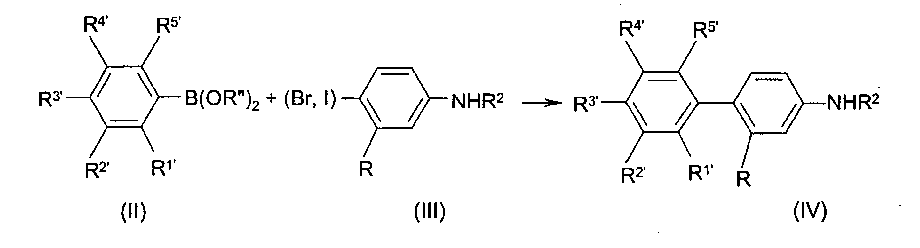
wobei R² Wasserstoff, Formyl oder Acyl mit 1 bis 5 C-Atomen (C(=O)-CₙH₂ₙ₊₁) (n=1-5) bedeutet und
R" für H, C₁-C₈-Alkyl steht oder B(OR")₂ ist ein Boronsäureanhydridrest;
gegebenenfalls anschließender Deacylierung zu (V) und nachfolgender Diazotierung zu Verbindungen der Formel (VI) und Umsetzung mit einem Nucleophil X, einem Cu-Salz nach Sandmeyer oder einem Reduktionsmittel zu 4-substituierten 2-Alkyl- und 2-Alkoxybiphenylen der Formel (I)

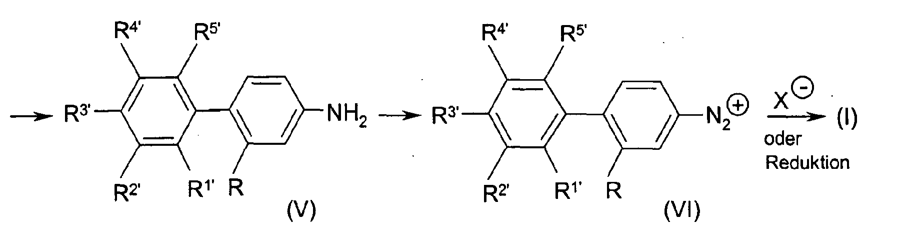

## Beschreibung

Die Erfindung betrifft ein Verfahren zur Herstellung von 4-substituierten 2-Alkyl- und 2-Alkoxybiphenylen.

Derartige 4-substituierte Biphenyle sind vielseitige Bausteine in der organischen Synthese und wichtige Zwischenprodukte in der Synthese von Wirkstoffen der agrochemischen und pharmazeutischen Industrie.

Trotz des bestehenden hohen wirtschaftlichen Interesses an diesen Verbindungen sind je nach konkretem Substitutionsmuster bisher entweder wenige und dann zudem teure und umständliche oder gar keine Herstellwege in der Literatur beschrieben.

Beispielsweise sind zu den Verbindungen 4-lod-2-methylbiphenyl, 4-Brom-2-methylbiphenyl, 4-Chlor-, 4-Brom- und 4-lod-2-methoxybiphenyl bisher keinerlei Synthesen in der chemischen Literatur dokumentiert.

Für 4-Chlor-2-methylbiphenyl sind verschiedene Synthesen beschrieben, die jedoch allesamt mit großen Nachteilen verbunden sind:
- So liefert beispielsweise die Bestrahlung von an sich schon schwierig darstellbarem 3-Chlor-2-methylbiphenyl in Cyclohexan (J. Chem. Soc. Perkin Trans. 2, 1983, 859-862) das Produkt im Gemisch mit Isomeren und Dehalogenierungsprodukten
- Ferner bekannt ist die Kupplung von 4-Chlor-1-iod-2-methylbenzol und lodbenzol mit Kupfer bei 270 °C (de la Mare, J. Chem. Soc. 1962, 3784-3796). Die Nachteile liegen insbesondere in den schlechten Selektivitäten und damit niedrigen Ausbeuten, sowie in der bereits aufwendigen Herstellung des Rohstoffs Chloriodmethylbenzol, dazu kommt, dass die so erhaltenen Produkte einer umständlichen Aufreinigung unterworfen werden müssen, um die Anforderungen für Pharmaqualitäten zu erfüllen.
- Ein weiteres bekanntes Verfahren ist die Diazotierung von 4-Amino-2-methylbiphenyl und Verkochen mit Benzol (Huntress, J. Am. Chem. Soc. 1939, 816, 820). Auch hier ist bereits die Herstellung des Rohstoffs Aminomethylbiphenyl sehr aufwendig; einen weiteren Nachteil stellt der Einsatz von cancerogenem Benzol dar. Außerdem sind auch hier die Ausbeuten nicht zufriedenstellend.

Es bestand daher das Bedürfnis ein Verfahren zur Herstellung von Verbindungen der Formel (I) zu entwickeln, das von kommerziell leicht erhältlichen und günstigen Ausgangsverbindungen ausgeht und die Zielprodukte in guten Ausbeuten und hohen Reinheiten zugänglich macht.

Die vorliegende Erfindung löst diese Aufgabe und betrifft ein Verfahren zur Herstellung von 4-substituierten 2-Alkyl- und 2-Alkoxybiphenylen der Formel (I), 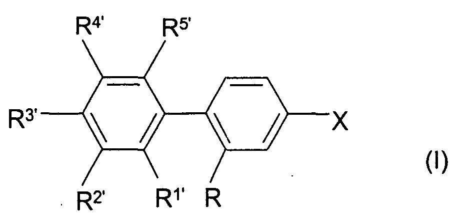 worin die Substituenten X, R und R^{1'} bis R^{5'} die folgende Bedeutung haben:
X steht für NH₂, NHAc, NH(C=O)R¹ mit R¹ = geradkettiges oder verzweigtes C₁-C₈-Alkyl, insbesondere C₁-C₄-Alkyl, Fluor, Chlor, Brom, lod, N₂H₃ oder Cyanid,
R steht für geradkettiges oder verzweigtes C₁- bis C₈-Alkyl, insbesondere C₁-C₄-Alkyl, oder C₁- bis C₅-Alkoxy, vorzugsweise C₁-C₃-Alkoxy, und
R^{1'} bis R^{5'} stehen unabhängig voneinander für H, CH₃, geradkettiges oder verzweigtes C₁-C₈-Alkyl, insbesondere C₁-C₄-Alkyl, F, Cl, CN, NO₂, CHO, COOH, CH₂OH, C(=O)CH₃, C₁-C₅-Alkoxy, insbesondere C₁-C₃-Alkoxy,
   durch Umsetzung von Phenylboronsäuren der Formel (II) mit 4-Brom- oder 4-lodalkyl- oder alkoxyanilinen oder -aniliden der Formel (III) zu Verbindungen der Formel (IV), 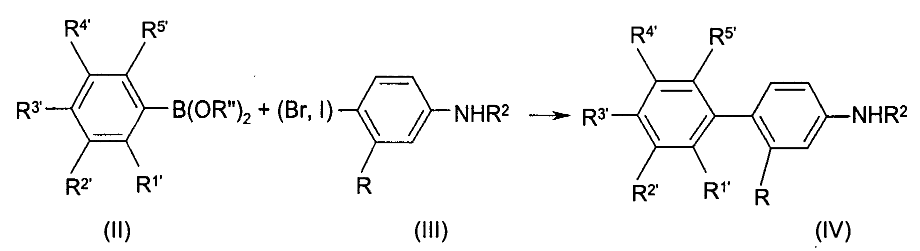
gegebenenfalls anschließender Deacylierung zu (V) und nachfolgender Diazotierung zu Verbindungen der Formel (VI) und Umsetzung mit einem Nucleophil X oder einem Reduktionsmittel zu 4-substituierten 2-Alkyl- und 2-Alkoxybiphenylen der Formel (I)

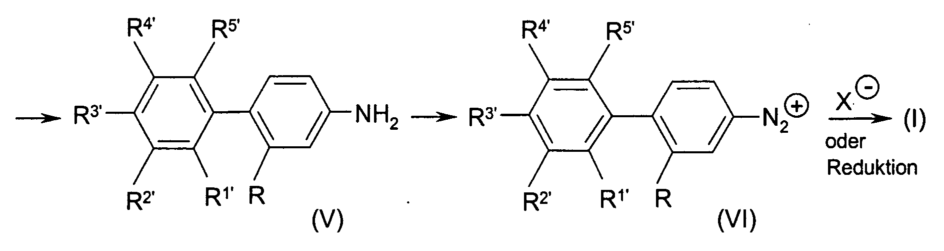

Das erfindungsgemäße Verfahren eröffnet einen effizienten, kurzen und mit auf jeder Stufe hohen Ausbeuten verbundenen Weg zum jeweiligen 4-substituierten 2-Alkyl-bzw. Alkoxybiphenyl (I).

In den voranstehenden Formeln (III) und (IV) kann R² Wasserstoff, Formyl oder Acyl mit n = 1 bis 5 C-Atomen (C(=O)-CₙH₂ₙ₊₁) sein.
Die Arylboronsäuren können als freie Boronsäuren (R" = H), als Boronsäureanhydride oder als Ester geradkettiger oder verzweigter, ein- oder mehrwertiger Alkohole eingesetzt werden (R" = C₁-C₈-Alkyl, insbesondere C₁-C₄-Alkyl-, oder B(OR")₂ ist ein Boronsäureanhydridradikal). Vorzugsweise werden die freien Boronsäuren, die Anhydride oder die Ester mit Methanol, Ethanol oder Glykol als Ausgangsverbindung eingesetzt.

Der Grund für den Umweg über acylierte Aniline und nachfolgende Deacylierung liegt darin, dass aufgrund der Labilität der NH₂-Gruppe in Kupplungsreaktionen in einigen Fällen mäßige Ausbeuten in der Kupplungsreaktion beobachtet werden. Wie überraschend gezeigt werden konnte, ist in diesen Fällen der genannte Umweg trotz höherer Stufenzahl aufgrund höherer Ausbeuten und Produktreinheiten ökonomischer.

Wie bereits oben beschrieben, kann in der voranstehenden Formel X die Bedeutungen NH₂, NHAc, NH(C=O)R¹ mit R¹ = geradkettiges oder verzweigtes C₁-C₈-Alkyl, Fluor, Chlor, Brom, lod, N₂H₃ oder Cyanid haben, die entsprechenden Reaktanden zur Herstellung dieser Verbindungen aus dem Diazoniumsalz sind dann entsprechend dem Stand der Technik beispielsweise CuBr für X = Br, Alkaliiodide für X = I, CuCN für X = CN, oder geeignete Reduktionsmittel wie z. B. Sulfite oder Disulfite oder ZnCl₂ für X = N₂H₃, um nur einige zu nennen .
Die Anilinderivate der Formel (V) werden nach Sandmeyer (Sandmeyerreaktion) über die entsprechenden Diazoverbindungen der Formel (VI) mit geeigneten Chlorid-, Bromid, lodid- oder Cyanidverbindungen zu 2-Alkyl- oder 2-Alkoxy-4-chlor-, brom-, iod- oder cyanobiphenylen der Formel (I) umgesetzt.
Die Herstellung von Hydrazinoverbindungen der Formel (I) (X= N₂H₃) erfolgt vorzugsweise durch Umsetzung der Diazoniumsalze der Formel (VI) mit einem Reduktionsmittel, insbesondere mit einem Sulfit, Hydrogensulfit, Disulfit oder Zinnchlorid.

Die Rohstoffe für die erfindungsgemäße Synthese sind entweder kommerziell erhältlich oder einfach und in guten Ausbeuten herstellbar. 4-Brom- oder 4-lod-3-alkyl- oder alkoxyaniline oder -anilide der Formel (III) sind z. B. allesamt auch in großen Mengen zu einem akzeptablen Preis käuflich erhältlich. Die für die Suzuki-Kupplungen einzusetzenden Phenylboronsäuren (II) sind ebenfalls kommerziell erhältlich, können aber in vielen Fällen in guten Ausbeuten kostengünstiger durch Umsetzung der entsprechenden Grignard-Verbindungen mit Borverbindungen nach dem Fachmann bekannten Methoden hergestellt werden.

Die Suzuki-Kupplungen von Boronsäuren der Formel (II) mit Anilinen der Formel (III) zu Aminobiphenylen der Formel (IV) gelingt durch Pd-katalysierte Kupplungsreaktionen, die in Anlehnung an Methoden des Standes der Technik durchführbar sind ("Metal-catalyzed Cross-coupling Reactions", Diederich/Stang, Wiley-VCH, Weinheim 1998). Für die Kupplungsreaktion kommen als Lösungsmittel beispielsweise Alkohole, DMSO, NMP, DMF, DMAc, Ether oder Kohlenwasserstoffe in Frage, bevorzugt ist die Kupplung in alkoholischen Lösungsmitteln, wie z.B. Methanol, Ethanol oder Glykol. Die Reaktion wird in Gegenwart von Edelmetallkatalysatoren, insbesondere von Palladium enthaltenden Katalysatoren, durchgeführt. In einer bevorzugten Ausführungsform werden Palladium- oder NickelKatalysatoren aus der folgenden Gruppe eingesetzt: NiCl₂, PdCl₂, PdCl₂(dppf), PdCl₂(PPh₃)₂, PdCl₂(dppe), PdCl₂(dppp), PdCl₂(dppb), Pdl₂, PdBr₂ oder Pd(OAc)₂ um nur einige zu nennen (darin sind dppe 1,2-Diphenylphosphinoethan, dppp-propan, dppb-butan und dppf 1,2-Bis(diphenylphosphino)ferrocen, Ph= Phenyl). In der Regel wird so vorgegangen, dass der Katalysator in dem entsprechenden Lösungsmittel vorgelegt wird und die Boronsäure der Formel (II) und das Anilin bzw. das Anilid der Formel (III) langsam zugetropft werden. Die Temperatur der Reaktionslösung wird während der Zugabe auf einer Temperatur im Bereich von 0 bis 150°C, insbesondere 70 bis 135°C gehalten Nach vollständiger Zugabe der Reaktanden kann es sinnvoll sein, 0,001 bis 1 Mol-% Katalysator, bezogen auf die Verbindung der Formel (II), noch einmal der Reaktionslösung zuzugeben, um einen möglichst vollständigen Umsatz zu erzielen. Anschließend wird die Lösung 1 bis 24 Stunden unter Rückfluss gekocht.

Nach vollständiger Kupplung wird die Reaktionsmischung beispielsweise durch Eintragen in Wasser, Extraktion und Eindampfen aufgearbeitet, wobei das Rohprodukt als erstarrte Schmelze oder viskoses Öl anfällt. In einigen Fällen kann das beim Eintragen in Wasser ausfallende Rohprodukt durch Filtration noch einfacher gewonnen werden.
Die Ausbeuten hierbei liegen im allgemeinen bei 85 bis 98 %, insbesondere 90 bis 95 %.

Die erhaltenen Rohprodukte können durch Umkristallisation unter Zusatz geringer Mengen Aktivkohle leicht und in guten Ausbeuten in hochreiner Form gewonnen werden. In den meisten Fällen erwies es sich jedoch als möglich, die rohen Biphenyle der Formel IV ohne weitere Aufreinigung einzusetzen.

Im Falle des Einsatzes von Anilinen mit R² = H erhält man direkt die für die Diazotierung erforderlichen Aniline (V) (R² = H); werden allerdings Anilide mit R² = Formyl oder Acyl mit 1 bis 5 C-Atomen (C(=O)-CₙH₂ₙ₊₁) (n=1-5) eingesetzt, so ist ein zusätzlicher Deacylierungsschritt erforderlich. Dieser kann vorteilhaft durch Kochen des rohen Anilids mit der später auch in der Diazotierungsstufe eingesetzten Säure, z. B. Salz-, Bromwasserstoff- oder Schwefelsäure, erfolgen. Diese Vorgehensweise bietet einen großen Vorteil, da nach Spaltung des Anilids direkt eine Lösung oder Suspension des Ammoniumsalzes resultiert, die direkt und ohne vorherige Freisetzung des Aminobiphenyls diazotiert werden kann (die als Nebenprodukt der Spaltung resultierenden Carbonsäuren stören nicht). Durch das meist mehrstündige Kochen der Anilide bzw. gebildeten Amine mit den Säuren fällt das Produkt zudem in sehr feiner Form an, so dass die Diazotierungen in sehr kurzen Reaktionszeiten quantitativ verlaufen.

Die weiteren Umsetzungen der gebildeten Diazoniumsalze zu den Produkten der Formel (I) werden in Anlehnung an Vorschriften des Standes der Technik durchgeführt, allerdings müssen diese oft in Anpassung an die bei Biphenylen häufig geringen Löslichkeiten geeignet modifiziert werden. Die Reduktion mit üblichen Reduktionsmitteln, wie z.B. Sulfit-, Hydrogensulfit- oder Disulfit-Lösungen oder SnCl₂ führt in guten Ausbeuten zu Hydrazinobiphenylen, die Umsetzung nach Sandmeyer mit CuCN, CuBr oder CuCI ergibt die entsprechenden Verbindungen mit X = CN, Br oder Cl in Ausbeuten zwischen 80 und 95 %, und die Umsetzung mit lodid-Lösungen führt in sehr guten Ausbeuten zu 4-Iod-2-alkyl/alkoxylbiphenylen.

Das erfindungsgemäße Verfahren soll durch die nachfolgenden Beispiele erläutert werden, ohne die Erfindung darauf zu beschränken:

### Beispiel 1

### Herstellung von 2-Methyl-4-acetaminobiphenyl

225 g Natriumcarbonat und 0,15 g PdCl₂(PPh₃)₂ in 450 ml Methanol werden auf Rückflusstemperatur erhitzt. Eine auf 50 °C erwärmte Lösung von 342 g 4-Brom-3-methylacetanilid und 270 g Phenylboronsäureethylenglykolester in 225 ml Methanol wird in 60 min zugetropft. Während der Dosierung wird jeweils so viel Methanol abdestilliert, dass die Innentemperatur auf jeweils mindestens 85 °C gehalten werden kann. Nach der vollständigen Zugabe werden weitere 0,1 g des Pd-Katalysators zugegeben und die Reaktionsmischung weitere 6 h am Rückfluss gekocht. Nach dieser Zeit zeigte die HPLC-Reaktionskontrolle einen Umsatz von 99,9 % an. Das verbleibende Methanol wird möglichst vollständig abdestilliert (620 ml), wobei die Reaktionsmischung noch rührbar bleiben muss. Anschließend werden 1250 ml Chlorbenzol zugegeben. Die Hydrolyse erfolgt durch Zugabe von 85 ml Wasser. Bei 70-80 °C werden die Phasen getrennt, die wässrige Phase wird verworfen. Die organische Phase wird einmal mit 50 ml Wasser gewaschen und anschließend über einen 0,5 cm dicke Schicht Aluminiumoxid filtriert. Das Chlorbenzol wird bei 300-500 mbar abdestilliert. Der verbleibende Rückstand von rohem 2-Methyl-4-acetaminobiphenyl wird ohne weitere Aufreinigung weiter eingesetzt. Die Ausbeute ist quantitativ.
Die direkte Kupplung des am Amino-Stickstoff nicht acylierten 4-Brom-3-methylanilins verlief mit Ausbeuten von nur etwa 55 %, der Rest wurde unter Deaminierung zu 2-Methylbiphenyl umgesetzt, welches zudem nicht einfach vom gewünschten Reaktionsprodukt abzutrennen war.

### Beispiel 2

### Herstellung von 2-Methyl-4-aminobiphenyl-Hydrochlorid

Zu dem wie in Beispiel 1 beschrieben hergestellten 2-Methyl-4-acetaminobiphenyl werden 300 g Wasser und 296 g HCI (37 %) zugegeben. Gegebenenfalls noch anhaftendes Chlorbenzol wird azeotrop abdestilliert, anschließend wird solange am Rückfluss gekocht, bis das HPLC-Monitorring vollständigen Umsatz anzeigt (etwa 4 h). Die erhaltene Suspension kann ohne weitere Reinigung und ohne Abtrennung der als Nebenprodukt der Spaltung entstandenen Essigsäure weiter umgesetzt werden. Die Ausbeute ist (aus HPLC-Flächenprozenten berechnet) 99 %.

### Beispiel 3

### Herstellung von 4-lod-2-methylbiphenyl

Die Reaktionsmischung aus Beispiel 2 wird mit 300 ml Wasser und 100 ml Toluol versetzt und auf 0 C abgekühlt. 517,5 g einer 20 Gew.-% Lösung von Natriumnitrit in Wasser werden in 2 h zwischen 0 und 3 °C zugetropft. Nach 15-minütigem Nachrühren wird eine Nitrit-Bestimmung durchgeführt. Sollte kein Nitrit mehr nachweisbar sein, werden jeweils weitere 2 g Nitritlösung zugegeben, 15 min nachgerührt und erneut Nitrit bestimmt. Wenn nach 15 min der Nitrit-Test noch positiv ist, ist der Umsatz vollständig, und unumgesetztes Nitrit kann durch Zusatz von 0,5 g Amidosulfonsäure vernichtet werden. Bei einer Temperatur von 0-5 °C werden insgesamt 516 g einer 50 %-igen Lösung von Kl in Wasser in 30 Minuten zugetropft. Zur Zerstörung von Resten unumgesetzten Diazoniumsalzes wird für 15 Minuten auf 60 °C erhitzt und anschließend wieder auf 50 °C abgekühlt. Nach Zugabe von 750 ml Toluol werden die Phasen getrennt und die wässrige noch einmal mit 250 ml Toluol extrahiert. Die vereinigten organischen Phasen werden durch Verrühren mit 189 g 2 M Na₂CO₃-Lösung, in der zusätzlich 11 g Natriumdisulfit gelöst sind, entsäuert und entfärbt. Nach erneuter Phasentrennung und Filtration der dunklen Lösung werden 455 g rohes 4-lod-2-methylbiphenyl als toluolische Lösung erhalten. Nach Abdestillieren des Toluols bei 33 mbar/60 °C verbleibt rohes 4-lod-2-methylbiphenyl als bräunliche, klare Flüssigkeit, die destillativ (allerdings durch partielle thermische Zersetzung mit Ausbeuteverlusten verbunden) in reines und farbloses, sehr lichtempfindliches 4-lod-2-methylbiphenyl überführt werden kann (b.p. 140-150 °C/2 mbar, Ausbeute an Rohprodukt 95 %, nach Destillation 90 %). Das Produkt muss bei 0-5 °C und lichtgeschützt aufbewahrt werden.

### Beispiel 4

### Herstellung von 4-Cyano-2-methylbiphenyl

Die wie in Beispiel 3 beschrieben hergestellte Suspension des Diazoniumsalzes wird mit einer frisch präparierten Lösung von CuCN in KCN-Lösung nach Vorschriften des Standes der Technik (Organikum, 17. Auflage, VEB 1988) umgesetzt. Man erhält 4-Cyano-2-methylbiphenyl als gelbliches Öl in einer Ausbeute von 83 %.

### Beispiel 5

### Herstellung von 4-Chlor-2-methylbiphenyl

Die wie in Beispiel 3 beschrieben hergestellte Suspension des Diazoniumsalzes wird mit einer frisch präparierten Lösung von CuCI in konzentrierter Salzsäure nach Vorschriften des Standes der Technik (Organikum, 17. Auflage, VEB 1988) umgesetzt. Man erhält 4-Chlor-2-methylbiphenyl als gelblichen Feststoff in einer Ausbeute von 86 %.

### Beispiel 6

### Herstellung von 4-Brom-2-methylbiphenyl

Die wie in Beispiel 3 beschrieben hergestellte Suspension des Diazoniumsalzes wird mit einer frisch präparierten Lösung von CuBr in konzentrierter Bromwasserstoffsäure nach Vorschriften des Standes der Technik (Organikum, 17. Auflage, VEB 1988) umgesetzt. Man erhält 4-Brom-2-methylbiphenyl als gelblichen Feststoff in einer Ausbeute von 92 %.

### Beispiel 7

### Herstellung von 4-Chlor-2-methylbiphenyl

Die wie in Beispiel 3 beschrieben hergestellte Suspension des Diazoniumsalzes wird mit einer frisch präparierten Lösung von CuCI in konzentrierter Salzsäure nach Vorschriften des Standes der Technik (Organikum, 17. Auflage, VEB 1988) umgesetzt. Man erhält 4-Chlor-2-methylbiphenyl als leicht bräunliches Öl in einer Ausbeute von 84 %.

### Beispiel 8

### Herstellung von 4-Hydrazino-2-methylbiphenyl

Die wie in Beispiel 3 beschrieben hergestellte Suspension des Diazoniumsalzes wird nach Vorschriften des Standes der Technik (Organikum, 17. Auflage, VEB 1988) mit Natriumsulfit-Lösung umgesetzt. Man erhält 4-Hydrazino-2-methylbiphenyl als gelblichen, sehr zersetzlichen und daher bei ―20 °C aufzubewahrenden oder sofort weiter umzusetzenden Feststoff. Das Hydrochlorid des Hydrazinobiphenyls ist bei Raumtemperatur und Luftausschluss einige Tage lang haltbar.

### Beispiel 9

### Herstellung von 4-Acetamino-2,4'-dimethylbiphenyl

Die Herstellung dieser Verbindung erfolgte analog zu Beispiel 1, wobei in diesem Fall die äquivalente Menge p-Tolylboronsäure-ethylenglykolester eingesetzt wurde. Nach dem Abdestillieren des Chlorbenzols verbleibt das Produkt als zähes, hellbraunes Öl in einer Ausbeute von 95 %, das durch Kristallisation aus Ethanol weiter aufgereinigt werden kann.

### Beispiel 10

### Herstellung von 4-Acetamino-2-methyl-4'-chlorbiphenyl

Die Herstellung dieser Verbindung erfolgte analog zu Beispiel 1, wobei in diesem Fall die äquivalente Menge p-Chlorphenylboronsäure-ethylenglykolester eingesetzt wurde. Nach dem Abdestillieren des Chlorbenzols verbleibt das Produkt als hellbrauner Feststoff.

## Patentansprüche

1. Verfahren zur Herstellung von 4-substituierten 2-Alkyl- und 2-Alkoxybiphenylen der Formel (I), 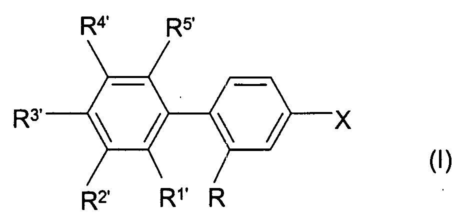 worin die Substituenten X, R und R^{1'} bis R^{5'} die folgende Bedeutung haben:
X steht für NH₂, NHAc, NH(C=O)R¹ mit R¹ = geradkettiges oder verzweigtes C₁- bis C₈-Alkyl, Fluor, Chlor, Brom, lod, N₂H₃ oder Cyanid,
R steht für geradkettiges oder verzweigtes C₁- bis C₈-Alkyl oder C₁- bis C₅-Alkoxy und
R^{1'} bis R^{5'} stehen unabhängig voneinander für H, CH₃, geradkettiges oder verzweigtes C₁- bis C₈-Alkyl, F, Cl, CN, NO₂, CHO, COOH, CH₂OH, C(=O)CH₃, C₁- bis C₅-Alkoxy, durch Umsetzung von Phenylboronsäuren der Formel (II) mit 4-Brom- oder 4-lodalkyl- oder alkoxyanilinen oder ―aniliden der Formel (III) zu Verbindungen der Formel (IV), 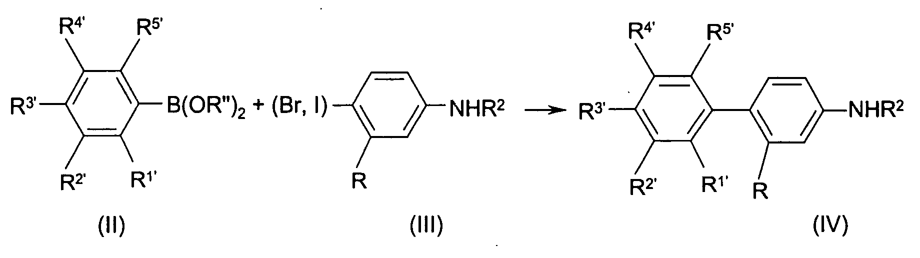
wobei R² Wasserstoff, Formyl oder Acyl mit 1 bis 5 C-Atomen (C(=O)-CₙH₂ₙ₊₁) (n=1-5) bedeutet und
R" für H, C₁-C₈-Alkyl steht oder B(OR")₂ ist ein Boronsäureanhydridrest; gegebenenfalls anschließender Deacylierung zu (V) und nachfolgender Diazotierung zu Verbindungen der Formel (VI) und Umsetzung mit einem Nucleophil X, einem Cu-Salz nach Sandmeyer oder einem Reduktionsmittel zu 4-substituierten 2-Alkyl- und 2-Alkoxybiphenylen der Formel (I).

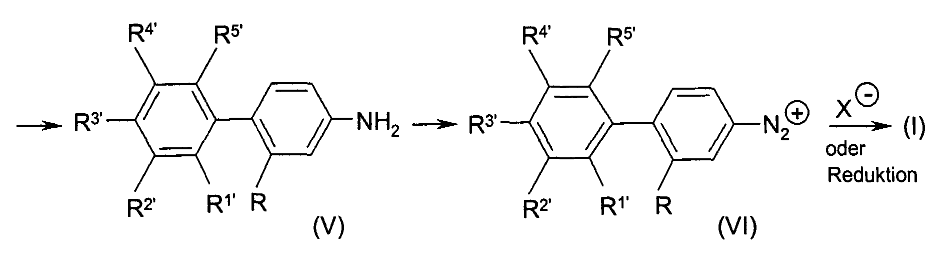

2. Verfahren nach Anspruch 1, **dadurch gekennzeichnet, dass** die Kupplungsreaktion der Phenylboronsäuren der Formel (II) mit Anilinen oder Aniliden der Formel (III) in einem alkoholischen Lösungsmittel durchgeführt wird.

3. Verfahren nach Anspruch 1 und/oder 2, **dadurch gekennzeichnet, dass** die Kupplungsreaktion in Gegenwart eines Edelmetallkatalysators durchgeführt wird.

4. Verfahren nach Anspruch 3, **dadurch gekennzeichnet, dass** ein Katalysator enthaltend Nickel oder Palladium eingesetzt wird.

5. Verfahren zur Herstellung von 4-substituierten 2-Alkyl- und 2-Alkoxybiphenylen der Formel (I), nach mindestens einem der vorhergehenden Ansprüche, **dadurch gekennzeichnet, dass** X= Cl, Br, I oder CN ist und Anilinderivate der Formel (V) über die entsprechenden Diazoverbindungen der Formel (VI) nach Sandmeyer mit geeigneten Chlorid-, Bromid, lodid- oder Cyanidverbindungen umgesetzt werden.

6. Verfahren zur Herstellung von 4-substituierten 2-Alkyl- und 2-Alkoxybiphenylen der Formel (I), nach mindestens einem der vorhergehenden Ansprüche, **dadurch gekennzeichnet, dass** X = N₂H₃ ist durch Reduktion des Diazoniumsalzes der Formel (VI) mit einem Reduktionsmittel, insbesondere mit einem Sulfit, Hydrogensulfit oder Disulfit oder Zinnchlorid.
